# EUROPEAN PATENT APPLICATION

(11) **EP 3 660 143 A2**
(43) Date of publication of application: **03.06.2020**
(21) Application number: 18838627.0
(22) Date of filing: 26.07.2018
(51) Int. Cl.: C12N 5/00, B01D 15/36

(54) **METHOD FOR ISOLATING EXTRACELLULAR VESICLE USING HYDROPHOBIC INTERACTION**

(30) Priority: 26.07.2017 KR 20170094874
(71) Applicant: Rosetta Exosome, Seoul 04385 (KR)
(72) Inventor: LEE, Chang Jin, Daegu 42038 (KR); GHO, Yong Song, Pohang-si Gyeongsangbuk-do 37673 (KR); PARK, Hyun Taek, Ulsan 44541 (KR)
(74) Representative: Berggren Oy, Tampere
(86) International application number: PCT/KR2018/008479
(87) International publication number: WO 2019/022538

(57) **Abstract**

The present invention relates to a method for isolating extracellular vesicles and, more particularly, to a method for isolating extracellular vesicles using hydrophobicity of the extracellular vesicles, and extracellular vesicles isolated using the method. When used, the method for isolating extracellular vesicles according to the present invention allows for isolating, from various animal body fluids including blood and urine or various tissues including cancer tissues, extracellular vesicles free of contamination with lipoproteins that are difficult to eliminate using a conventional method, can solve conventional problems caused by lipoprotein contamination, and is expected to be an essential technology that has a great influence on various research using extracellular vesicles isolated from various animal body fluids or tissues, such as characterization and function research, multi-omics research, excavation of novel biomarkers, diagnosis and treatment, etc.

## Description

### Technical Field

The present invention relates to a method for isolation of extracellular vesicles and, more particularly, to a method for isolating extracellular vesicles by using the hydrophobicity thereof and to extracellular vesicles, free of impurities including lipoproteins, isolated by the method.

### Background Art

Extracellular vesicles are nano-sized particles (20-100,000 nm) that are naturally released from all cells on the earth and have cell-derived lipid bilayer membrane structures. For animals, extracellular vesicles are found in body fluid such as blood, saliva, tear, nasal mucus, sweat, urine, feces, cerebrospinal fluid (CSF), ascite, amniotic fluid, bronchoalveolar lavage fluid, pleural effusion, semen, milk, etc. as well as various tumors or normal tissues.

Consisting of various physiologically active materials, such as proteins, lipids, amino acids, RNA, and the like, from the secreting cells (mother cells), extracellular vesicles represent physiological and pathological traits of the mother cells. In addition, extracellular vesicles have been found to perform various physiological and pathological functions as carriers that combine with other cells and tissues to transfer physiologically active materials from the mother cells to the recipient cells and tissues. Accordingly, studies on constituents and functions of extracellular vesicles have been ongoing with the expectation of utilizing extracellular vesicles as a new approach for diagnosing and treating various diseases.

Conventionally, many techniques for isolating extracellular vesicles have been used, including ultracentrifugation, density gradient ultracentrifugation, sonication, size exclusion, polymer-based precipitation, salt-based precipitation, organic solvent-based precipitation, ion exchange chromatography, affinity chromatography, and aqueous two phase system. Of them, ultracentrifugation is most widely employed. The techniques are effective for isolating nanoparticles from relatively small materials and thus are applied to the separation of extracellular vesicles from various materials including soluble proteins within samples.

With respect to the utilization of extracellular vesicles, there are ongoing studies in which extracellular vesicles separated from in vitro cultured cells are analyzed for constituents with the aim of excavating novel biomarkers as well as studies in which extracellular vesicles separated from animal body fluid including blood or various tissues including cancer tissues are used for quantitative comparison of constituents such as proteins, nucleic acids, and the like, thereby availing the extracellular vesicles in many aspects including diagnosis, prognosis prediction, or treatment of diseases.

Particularly, there is a great demand on diagnosis and therapy using extracellular vesicles derived from various animal body fluid including blood (serum and plasma) and urine and various tissues including cancer tissues. However, because nanoparticles present in animal body fluid or tissues include various forms of lipoproteins as well as extracellular vesicles, it is difficult to selectively isolate only extracellular vesicles with conventional extracellular vesicle isolation techniques.

A lipoprotein is an important factor for transporting cholesterol and triglycerides, which are building blocks to create membranes essential for cell constitution. In addition, a lipoprotein is known to serve as a carrier for transporting extracellular nucleic acids within blood (Nat Cell Biol, 2011, Vicker et al., ISSN: 1476-4679), closely resembling extracellular vesicles that function to transport extracellular nucleic acids within blood. According to sizes (nm) and densities (g/mL), lipoproteins are divided into the following nanoparticles: high-density lipoprotein (HDL; 5-15 nm, 1.063 g/mL), low-density lipoprotein (LDL; 18-28 nm, 1.019-1.063 g/mL), intermediate-density lipoprotein (IDL; 25-50 nm, 1.006-1.019 g/mL), very low-density lipoprotein (VLDL; 30- 80 nm, 0.950-1.006 g/mL), and chylomicron (100-1000 nm, about 0.95 g/mL).

Such extracellular vesicles and lipoproteins within blood are similar to each other in terms of various aspect including size and density in addition to the function of transporting extracellular nucleic acids and thus difficult to selectively separate from each other (Biochem Biophys Res Commun, 2009, Looze et al., ISSN: 1090-2104; J Extracell Vesicles, 2014, Yuana et al., ISSN: 2001-3078; Sci Rep, 2016, Sodar et al., ISSN: 2045-2322).

Hence, the extracellular vesicles isolated from various animal body fluids including blood and urine and various tissues including cancer tissues are contaminated with lipoproteins and cause various problems when used in genomes and protein body analysis, functional search, and application to diagnosis and therapy. It has been reported that lipoproteins cannot be effectively removed even by density gradient ultracentrifugation that takes advantage of a density difference between extracellular vesicles and lipoproteins.

Therefore, there is an urgent need for developing a technique for effectively removing lipoproteins upon the isolation of extracellular vesicles in order for extracellular vesicles derived from various animal body fluids or tissues to be used in research into the analysis and function thereof as well as application to diagnosis and therapy. Together with the isolation of extracellular vesicles, the effective removal of lipoproteins would be one of the most critical techniques to the development of the art.

### Detailed Description of the Invention

### Technical Problem

An aspect of the present invention is to provide a method for isolating extracellular vesicles, the method comprising the steps of: (a) immobilizing a hydrophobic functional group onto a stationary phase; (b) loading a sample containing extracellular vesicles to the stationary phase; (c) washing out remaining sample residues not bound to the hydrophobic functional group on the stationary phase; and (d) eluting extracellular vesicles bound to the hydrophobic functional group from the stationary phase.

Another aspect of the present invention is to provide a method for isolating extracellular vesicles, the method comprising the steps of: (a) immobilizing a hydrophobic functional group onto a stationary phase; (b) loading a sample including extracellular vesicles to the stationary phase; and (c) collecting a sample fraction containing extracellular vesicles not bound to the hydrophobic functional group from the stationary phase.

Another aspect of the present invention is to provide extracellular vesicles isolated by the method.

Another aspect of the present invention is to provide a method for removing lipoproteins from the sample fraction containing extracellular vesicles, by using the above isolation method.

### Technical Solution

The present invention has been made to solve the above-mentioned problems, and an aspect of the present invention is to provide a method for specifically isolating extracellular vesicles from materials higher or lower in hydrophobicity than extracellular vesicles by using the hydrophobic interaction of extracellular vesicles. In the present invention, when various samples containing extracellular vesicles are allowed to pass through a hydrophobic functional group-bound stationary phase under the control of salting-out ions, the extracellular vesicles can be rapidly and easily isolated according to the hydrophobic interaction thereof without physical and chemical changes.

As used herein, the term "extracellular vesicles" refers collectively to biological nanoparticles derived from cells of Archaea, Prokarya, or Eukarya. Examples of extracellular vesicles include argosomes, dexosomes, exosomes, ectosomes, exovesicles, oncosomes, prominosome, prostasomes, tolerosomes, microparticles, microvesicles, nanovesicles, blebbing vesicles, budding vesicles, exosome-like vesicles, matrix vesicles, membrane vesicles, shedding vesicles, membrane particles, shedding microvesicles, membrane blebs, epididimosomes, promininosomes, texosomes, and archeosomes, but are not limited thereto. In addition, various kinds of extracellular vesicle mimics including various membrane proteins also fall within the scope of the extracellular vesicles.

In detail, the first method for isolating extracellular vesicles according to the present invention comprises the steps of: (a) immobilizing a hydrophobic functional group onto a stationary phase; (b) loading a sample including extracellular vesicles to the stationary phase; (c) washing out remaining sample residues not bound to the hydrophobic functional group on the stationary phase; and (d) eluting extracellular vesicles bound to the hydrophobic functional group from the stationary phase.

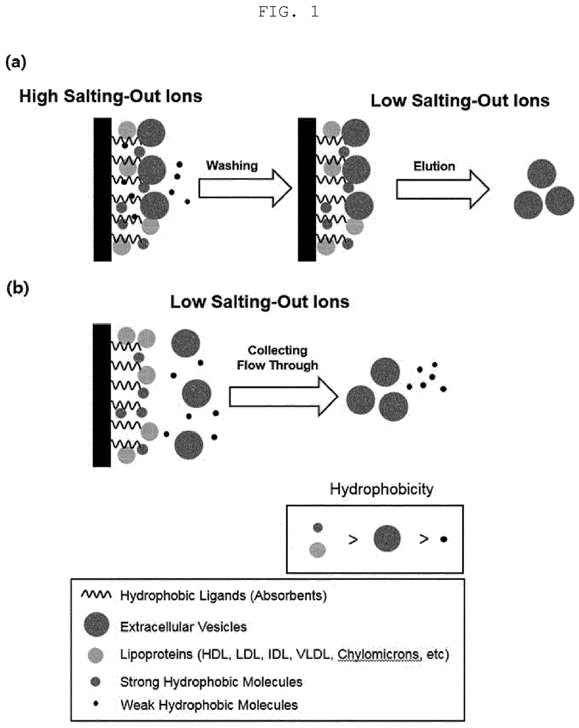

The extracellular vesicle isolating method according to the present invention is schematically illustrated in FIG. 1A.

The second method for isolation of extracellular vesicles according to the present invention comprises the steps of: (a) immobilizing a hydrophobic functional group onto a stationary phase; (b) loading a sample containing extracellular vesicles to the stationary phase; and (c) collecting a sample fraction including extracellular vesicles not bound to the hydrophobic functional group from the stationary phase.

The extracellular vesicle isolating method according to the present invention is schematically illustrated in FIG. 1B.

As used herein, the term "stationary phase" refers to a substrate for immobilizing thereto one of two substances that exhibit physical, chemical, or biological affinity in affinity chromatography. In detail, the stationary phase in the present invention may be selected from the group, but not limited to, agarose beads, sepharose beads, sephadex beads, silica beads, magnetic beads, gold nanoparticles, silver nanoparticles, iron oxide nanoparticles, a nylon membrane, nitrocellulose membrane, a PVDF membrane, paper, plastic, glass, and a metal sensor chip.

As used herein, the term "hydrophobic functional group", also called hydrophobic working group or an apolar functional group, refers to a molecule having no or almost no affinity for water molecules or to a functional group the surface of which is not dissolved in water or repels water therefrom, as accounted for by alkyl, phenyl, etc. Particularly, the hydrophobic functional group of the present invention may be selected from the group consisting of butyl, cyanobutyl, octyl, phenyl, and diphenyl, but is not limited thereto.

The term "sample", as used herein, is intended to encompass a biological sample, a cell culture, or a tissue sample containing extracellular vesicles therein. Concrete examples of the sample include a mammalian cell culture, a bacterial cell culture, a yeast culture, a tissue extract, a cancer tissue, blood (serum and plasma), saliva, tear, nasal mucus, sweat, urine, feces, cerebrospinal fluid (CSF), ascite, amniotic fluid, bronchoalveolar lavage fluid, pleural effusion, semen, milk, dust, fresh water, seawater, soil, and fermented foods, but are not limited thereto.

The term "solution", as used herein, refers to a liquid in which extracellular vesicles are or are not contained. The liquid may be used to wash out the stationary phase before or after sample loading or to elute a sample bound to the stationary phase. The solution of the present invention may be selected from the group consisting of water, an organic solvent, and a buffer, but is not limited thereto.

As used herein, the term "salting-out ion" refers to a kosmotropic salt for stabilizing the structure of water, which is an ion that reduces the availability of water in a solution to increase the intensity of hydrophobic interaction. A scale for such kosmotropic salts can be established according to the Hofmeister series in order of their ability to effect the solubility of a soluble material in a solution. Anions are usually ordered as follows: SO₄²⁻ < HPO₄²⁻ < OH⁻ < F⁻ < HCOO⁻ < CH₃COO⁻ < Cl⁻ < Br⁻ < NO₃⁻ < I⁻ < SCN⁻ < ClO₄⁻. The order of cations is usually given as: NH₄⁺, Rb⁺, K⁺, Na⁺, Cs⁺, Li⁺, Ca²⁺, Mg²⁺, and Ba²⁺. Kosmotropic salts act as salting-out ions for hydrophobic particles according to the Hofmeister series. The salting-out ions of the present invention may be a kosmotropic salt, composed of an anion and a counter ions thereof in the Hofmeister series, for stabilizing the structure of water.

Binding of extracellular vesicles in a sample to the hydrophobic functional group on the stationary phase may be determined by controlling kinds and concentrations of the salting-out ions.

In detail, when extracellular vesicles are isolated from a material which is weaker in intensity of hydrophobic interaction than extracellular vesicles in a sample, the stationary phase having a hydrophobic functional group thereon is washed with a solution containing a high concentration of salting-out ions, followed by flowing an extracellular vesicle sample added with a high concentration of salting-out ions through the stationary phase. As a result, the extracellular vesicles are adsorbed onto the stationary phase having the hydrophobic functional group thereon while particles with a relatively weak intensity of hydrophobic interaction pass out of the column without being bound thereto. Subsequently, a solution containing salting-out ions at a concentration lower than that of the salting-out ions mixed in the stationary phase washing solution and the sample is used to wash the material lower in hydrophobic interaction than the extracellular vesicles out of the stationary phase. Thereafter, the extracellular vesicles adsorbed onto the stationary phase can be separated by elution in a conventional approach using water or a solution containing no salting-out ions or a low concentration of salting-out ions.

By contrast, when extracellular vesicles are isolated from a material which is stronger in intensity of hydrophobic interaction than extracellular vesicles in a sample, the stationary phase having a hydrophobic functional group thereon is washed with water or a solution containing a low concentration of salting-out ions. As a result, the material with a relatively strong intensity of hydrophobic interaction is adsorbed to the stationary phase while the extracellular vesicles pass out of the column without being bound thereto. If necessary, a lower concentration of the salting-out ions in the sample containing extracellular vesicles may more effectively separate extracellular vesicles that are not absorbed onto the stationary phase having a hydrophobic functional group thereon.

In the present invention, the salting-out ions may be selected from the group consisting of kosmotropic salts. Examples of the kosmotropic salts include, but are not limited to, sulphate, phosphate, hydroxide, fluoride, formate, acetate, chloride, bromide, nitrate, iodide, thiocyanate, citrate, and tartrate, together with the counter cations thereof. Particularly, the salting-out ions of the present invention may be selected from the group consisting of sodium chloride, ammonium sulfate, sodium sulfate, and ammonium chloride, but is not limited thereto.

Provided according to the present invention is a method for isolation of extracellular vesicles from various samples by controlling concentrations of a salting out-ion, in which 1) extracellular vesicles are bound to a stationary phase having a hydrophobic functional group thereon and materials with low intensity of hydrophobic interaction are removed, followed by eluting the bound extracellular vesicles to separate extracellular vesicles from materials different therefrom in terms of the intensity of hydrophobic interaction, or 2) materials with high intensity of hydrophobic interaction are adsorbed onto a stationary phase having a hydrophobic functional group thereon while extracellular vesicles are eluted in a non-binding manner. By the method, extracellular vesicles can be isolated from various samples fast and easily, and the extracellular vesicles thus obtained can find applications in various fields including diagnosis and therapy and multi-omics and in research into characterization and functions of target materials of interest.

The present invention provides two approaches into the isolation of extracellular vesicles by using the hydrophobic interaction.

First, the present invention provides a method for isolation of extracellular vesicles from materials smaller in intensity of hydrophobic interaction than the extracellular vesicles, the method comprising the steps of: (a) immobilizing a hydrophobic functional group onto a stationary phase; (b) loading a sample containing extracellular vesicles to the stationary phase to bind the extracellular vesicles to the stationary phase; (c) washing out remaining sample residues not bound to the hydrophobic functional group on the stationary phase; and (d) eluting extracellular vesicles bound to the hydrophobic functional group from the stationary phase.

In an embodiment of the present invention, the method may further comprise a step of washing the stationary phase having the hydrophobic function group thereon with a solution containing salting-out ions, prior to step (b).

In this regard, the salting-out ions in the stationary phase-washing step have a concentration suitable to make the stationary phase strong in hydrophobicity to enable the extracellular vesicles to bind to the stationary phase. The concentration of the salting-out ions may vary depending on kinds of the salting-out ions in the washing solution and the intensity of hydrophobicity of the stationary phase before the washing. Through the stationary phase washing step, the extracellular vesicles are bound to the stationary phase while nanoparticles that are lower in hydrophobicity than the extracellular vesicles according to the concentration of the salting-out ions can pass through the stationary phase as they are. Particularly, the concentration of the salting-out ions in the present invention may be controlled to be within 0 M to 10 M.

In an embodiment of the present disclosure, the method may further comprise a step of adding a solution containing salting-out ions to the sample to change kinds and concentrations of salting-out ions contained in the sample, prior to step (b) of loading a sample containing extracellular vesicles to the stationary phase.

By adding a solution containing salting-out ions to the sample, the salting-out ions contained in the sample may be increased or decreased in concentration or may change or be supplemented in kind. As such, kinds or concentrations of salting-out ions contained in the sample are adjusted to control the intensity of hydrophobic interaction of the sample, which leads to controlling the hydrophobic interaction between the extracellular vesicles and the stationary phase. Particularly, the concentration of the salting-out ions may be controlled to be within the range of 0 M to 10 M.

In an embodiment of the present invention, step (d) is to specifically separate extracellular vesicles from materials adsorbed onto the stationary phase having a hydrophobic functional group and the method may further comprise a step of controlling the kind or concentration of the salting-out ions to remove materials weaker in intensity of hydrophobic interaction than the extracellular vesicles, prior to step (d) of eluting the extracellular vesicles.

In this regard, the concentration of the salting-out ions is in a level which allows the elution of only materials weaker in hydrophobicity than the extracellular vesicles while the extracellular vesicles are remaining bound to the stationary phase. The concentration of the salting-out ions may be controlled depending on kinds of the salting-out ions and pH or temperatures of the solution. Particularly, the concentration of the salting-out ions may be controlled to be within the range of 0 M to 10 M.

In an embodiment of the present invention, step (d) is to specifically separate the extracellular vesicles from the materials adsorbed to the stationary phase having a hydrophobic functional group thereon. In this step, the salting-out ions may be controlled with respect to kind or concentration so as to elute only the extracellular vesicles while the materials greater in hydrophobicity than the extracellular vesicles are remaining bound to the column.

In this regard, the concentration of the salting-out ions is in a level which allows the elution of only the extracellular vesicles while the materials greater in hydrophobicity than the extracellular vesicles are remaining bound to the stationary phase. The concentration of the salting-out ions may be controlled depending on kinds of the salting-out ions and pH or temperatures of the solution. Particularly, the concentration of the salting-out ions may be controlled to be within the range of 0 M to 10 M.

The salting-out ions of the present invention are used to control the hydrophobicity of the stationary phase or the sample containing the extracellular vesicles. In the present invention, various conditions understood to those skilled in the art can be applied in order to bind extracellular vesicles to the stationary phase or to elute extracellular vesicles bound to the stationary phase. In detail, the hydrophobicity of the stationary phase may be controlled with the kind and concentration of the salting-out ions and the pH and temperature of the buffer used. Particularly, the salting-out ions contained in the solution are at least one selected from the group consisting of sulphate, phosphate, hydroxide, fluoride, formate, acetate, chloride, bromide, nitrate, iodide, thiocyanate, citrate, and tartrate. A condition applicable to the solution include a salting-out ion concentration of 0 M-10 M, a pH of 3-12, a temperature of 4°C-50°C, or a combination thereof, but is not limited thereto.

In the present invention, the hydrophobic functional group or the sample may be loaded to the stationary phase in a gravity-, pumping- (liquid chromatography system), injection-, rotation-, or vacuum-type manner, but without limitations thereto.

The method for isolation of extracellular vesicles according to the present invention may further comprise a step of pre-treating the sample containing the extracellular vesicles, prior to the step of loading the sample to the stationary phase.

The pre-treatment step of the present invention is a step of partially purifying an unpurified sample and may be conducted by a technique selected from, but not limited to, centrifugation, ultracentrifugation, filtration, ultrafiltration, dialysis, desalting column chromatography, sonication, density gradient ultracentrifugation, size exclusion chromatography, ion exchange chromatography, affinity chromatography, polymer-based precipitation, salt-based precipitation, organic solvent precipitation, and an aqueous two-phase system.

In addition, the method for isolating extracellular vesicles according to the present invention may further comprise a step of post-treating the extracellular vesicles eluted from the stationary phase having the hydrophobic functional group immobilized thereonto.

The post-treatment step of the present invention is to a step of purifying the separated extracellular vesicles and may be conducted by a technique selected from centrifugation, ultracentrifugation, filtration, ultrafiltration, dialysis, desalting column chromatography, sonication, density gradient ultracentrifugation, size exclusion chromatography, ion exchange chromatography, affinity chromatography, salt-based precipitation, organic solvent precipitation, and an aqueous two-phase system, but without limitations thereto.

Second, the present invention provides a method for isolating extracellular vesicles from materials greater in intensity of hydrophobic interaction than the extracellular vesicles, the method comprising the steps of: (a) immobilizing a hydrophobic functional group onto a stationary phase; (b) loading a sample including extracellular vesicles to the stationary phase; and (c) collecting a sample fraction containing extracellular vesicles not bound to the hydrophobic functional group from the stationary phase.

In an embodiment of the present invention, the method may further comprise a step of washing the stationary phase having hydrophobic functional group immobilized thereon with a solution containing salting-out ions, prior to step (b).

In this regard, the salting-out ions in the stationary phase-washing step have a concentration suitable to make the stationary phase weak in hydrophobicity so as to allow only the material stronger in hydrophobicity than the extracellular vesicles to bind to the stationary phase, with no permissions of binding the extracellular vesicles to the stationary phase. The concentration of the salting-out ions may vary depending on kinds of the salting-out ions in the washing solution and the intensity of hydrophobicity of the stationary phase before the washing. Through the stationary phase washing step, materials stronger in hydrophobicity than the extracellular vesicles are bound to the stationary phase while the extracellular vesicles and the nanoparticles that are weaker in hydrophobicity than the extracellular vesicles can pass through the stationary phase as they are. Particularly, the concentration of the salting-out ions in the present invention may be controlled to be within 0 M to 10 M.

In an embodiment of the present invention, the method may further comprise a step of adding a solution either containing or not containing salting-out ions to the sample to change kinds or concentrations of salting-out ions contained in the sample, prior to step (b) of loading a sample containing extracellular vesicles to the stationary phase.

By adding a solution containing or not containing salting-out ions to the sample, the salting-out ions contained in the sample may be increased or decreased in concentration or may change or be supplemented in kind. As such, kinds or concentrations of salting-out ions contained in the sample are adjusted to control the intensity of hydrophobic interaction of the sample, which leads to controlling the hydrophobic interaction between the extracellular vesicles and the stationary phase. Particularly, the concentration of the salting-out ions may be controlled to be within the range of 0 M to10 M.

The solution containing or not containing salting-out ions of the present invention are used to control the hydrophobicity of the stationary phase or the sample containing the extracellular vesicles. In the present invention, various conditions understood to those skilled in the art can be applied in order to selectively adsorb only materials, such as lipoproteins, stronger in hydrophobicity than the extracellular vesicles, to the stationary phase having a hydrophobic functional group thereon while extracellular vesicles having a relatively weak intensity of hydrophobic interaction pass as they are, without being adsorbed to the stationary phase. In detail, the hydrophobicity of the stationary phase may be controlled with the kind and concentration of the salting-out ions and the pH and temperature of the buffer used. Particularly, the salting-out ions contained in the solution are at least one selected from the group consisting of sulphate, phosphate, hydroxide, fluoride, formate, acetate, chloride, bromide, nitrate, iodide, thiocyanate, citrate, and tartrate. A condition applicable to the solution include a salting-out ion concentration of 0 M-10 M, a pH of 3-12, a temperature of 4°C-50°C, or a combination thereof, but is not limited thereto.

Having high intensities of hydrophobic interaction, lipoproteins in biological samples including blood are known to strongly bind to a hydrophobic functional group even in the environment of low salting-out ion concentrations. Accordingly, the method of the present invention can effectively separate such lipoproteins and extracellular vesicles from biological samples.

In the present invention, the hydrophobic functional group or the sample may be loaded to the stationary phase in a gravity-, pumping- (liquid chromatography system), injection-, rotation-, or vacuum-type manner, but without limitations thereto.

The method for isolation of extracellular vesicles according to the present invention may further comprise a step of pre-treating the sample containing the extracellular vesicles, prior to the step of loading the sample to the stationary phase.

The pre-treatment step of the present invention is a step of partially purifying an unpurified sample and may be conducted by a technique selected from, but not limited to, centrifugation, ultracentrifugation, filtration, ultrafiltration, dialysis, desalting column chromatography, sonication, density gradient ultracentrifugation, size exclusion chromatography, ion exchange chromatography, affinity chromatography, polymer-based precipitation, salt-based precipitation, organic solvent precipitation, and an aqueous two-phase system.

In addition, the method for isolation of extracellular vesicles according to the present invention may further comprise a step of post-treating the extracellular vesicles eluted from the stationary phase having the hydrophobic functional group immobilized thereon.

The post-treatment step of the present invention is to a step of purifying the separated extracellular vesicles and may be conducted by a technique selected from centrifugation, ultracentrifugation, filtration, ultrafiltration, dialysis, desalting column chromatography, sonication, density gradient ultracentrifugation, size exclusion chromatography, ion exchange chromatography, affinity chromatography, salt-based precipitation, organic solvent precipitation, and an aqueous two-phase system, but without limitations thereto.

Furthermore, the present invention provides the extracellular vesicles isolated by the isolation method.

In addition, the present invention provide a method for effectively removing a lipoprotein stronger in intensity of hydrophobic interaction than extracellular vesicles or a material weaker in intensity of hydrophobic interaction than extracellular vesicles from a sample containing the extracellular vesicles by using the isolation method.

### Advantageous Effects

The method for isolation of extracellular vesicles according to the present invention does neither require an expensive apparatus, such as a centrifugal separator, nor allow the sample to be exposed to an extreme environment during the isolation procedure thereof, thus enjoying the advantage of effectively isolating extracellular vesicles without changing morphologies and characteristics thereof. In addition, the method of the present invention can be applied before or after conducting a conventional method, with the resultant maximization of isolation efficiency.

Furthermore, the method for isolation of extracellular vesicles according to the present invention can not only isolate extracellular vesicles fast and effectively without limitations to the quantity of samples, thus playing an important role in the mass production of extracellular vesicles, but also be used in clinical diagnosis by being applied to pre- and/or post-treatment of a small amount of body fluid samples.

The use of the method for isolation of extracellular vesicles according to the present invention enables the separation of extracellular vesicles free of contamination with lipoproteins from various animal body fluids including blood and urine or various tissues including cancer tissues, which is difficult to achieve by conventional methods, thus solving conventional problems with lipoprotein contamination. Also, the method of the present invention is expected to be an essential technique having a great influence on various studies on characteristics and functions of extracellular vesicles isolated from various animal body fluids or tissues, multi-omics, excavation of novel biomarkers, diagnosis and therapy, and the like.

### Brief Description of the Drawings

FIG. 1 shows schematic views illustrating a method for isolation of extracellular vesicles according to an embodiment of the present invention.
FIG. 2 is a conceptual view illustrating an overall scheme for a method for isolation of reference extracellular vesicles from the colon cancer cell line SW480 according to an embodiment of the present invention.
FIG. 3 shows the identification of reference extracellular vesicles isolated according to an embodiment of the present invention, as analyzed by size exclusion chromatography (a), transmission electron microscopy (b), DLS analysis (c), and Western blotting (d) .
FIG. 4 shows the binding behavior of reference extracellular vesicles to a stationary phase having a hydrophobic functional group immobilized thereonto according to an embodiment of the present invention, as identified by nanoparticle concentration analysis (a) and sandwich ELISA analysis (b).
FIG. 5 shows the binding behavior of reference extracellular vesicles to a stationary phase having a hydrophobic functional group immobilized thereonto in the environment of various concentrations of sodium chloride according to an embodiment of the present invention, as identified by nanoparticle concentration analysis (a) and Western blotting (b).
FIG. 6 shows the purification of human plasma-derived extracellular vesicles being removed of lipoproteins therefrom through an isolation method using hydrophobic interaction according to an embodiment, as identified by Western blotting (a), cholesterol assay (b), nanoparticle concentration analysis (c), and protein concentration analysis (d).

### Best Mode for Invention

### Mode for Carrying Out the Invention

Hereinafter, the present invention will be described in detail with reference to examples. These examples are only for illustrating the present invention more specifically, and it will be apparent to those skilled in the art that the scope of the present invention is not limited by these examples.

### EXAMPLE 1: Purification and Analysis of Reference Extracellular Vesicles

A fresh culture of the colon cancer cell line SW480 was centrifuged at 500 xg for 10 min (repeated twice in total). After removal of the cell debris and the precipitates, the supernatant was centrifuged again at 2,000 xg for 20 min (repeated twice in total) and the precipitate was removed.

The supernatant was added with a precipitation-inducing agent (8.4% Polyethylene Glycol 6000, 250 mM NaCl, 20 mM HEPES, pH7.4) in order to primarily purify and precipitate extracellular vesicles existing therein. After being stored for 16 hours in a refrigerator, the supernatant was centrifuged at 12,000 xg for 30 min to harvest extracellular vesicles as a precipitate. The precipitate was dissolved in HEPES buffer (HEPES-buffered saline, 20 mM HEPES, 150 mM NaCl, pH 7.4).

In order to secondarily purify the extracellular vesicles by use of density and buoyancy, the sample was mixed with OptiPrep™ (final concentration 30%) and the mixture was loaded to the bottom of an ultracentrifugation vessel, followed by layering 20% OptiPrep™ and 5% OptiPrep™ thereon in the order. After buoyant density gradient ultracentrifugation at 200,000 xg for 2 hours (30%, 20%, and 5% OptiPrep™ triple layers), extracellular vesicles and a high-density (1.08-1.12 g/ml) area were harvested.

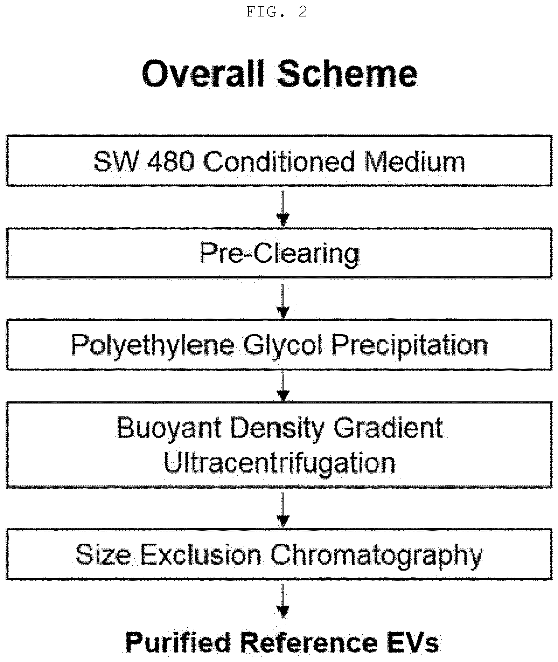

For tertiary purification of the purified extracellular vesicles, size exclusion chromatography was performed on a Sephacryl S500-filled column (10 x 100 mm) into which the harvest was previously loaded with the aid of an HPLC apparatus. As a result, a fraction of finally purified extracellular vesicles was obtained. This isolation procedure of sample extracellular vesicles is illustrated in FIG. 2.

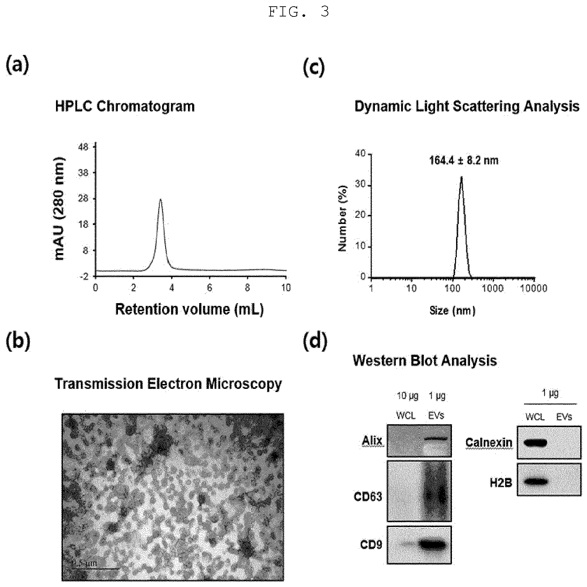

The extracellular vesicles finally purified, as described above, from the colon cancer cell line SW480 were identified using size exclusion chromatography, electron microscopy, and Dynamic Light Scattering (DLS). The results are depicted in FIG. 3. As shown in FIG. 3 (a), the colon cancer cell line-derived extracellular vesicles were found to be highly pure. In addition, the colon cancer cell line-derived extracellular vesicles were morphologically observed under a transmission electron microscope as shown in FIG. 3(b) and were analyzed to have a diameter of about 164 nm as measured by DLS in FIG. 3(c).

Furthermore, as shown in FIG. 3(d), Western blot analysis indicated selectively high expression levels of Alix, CD63, and CD9 proteins, which are markers of extracellular vesicles, compared with the cell line, but did not detect Calnexin and histone H2B, which are cytosolic proteins, in the purified extracellular vesicles derived from the colon cancer cell line.

### EXAMPLE 2: Binding Behavior of Reference Extracellular Vesicles to Stationary Phase Having Hydrophobic Functional Group

Examination was made to see whether or not extracellular vesicles bind to a hydrophobic functional group. In this regard, an HPLC system was used to examine the binding behavior of the reference extracellular vesicles separated in Example 1.

First, a column (1 ml) filled with a stationary phase (Phenyl-Sepharose) having a hydrophobic functional group was washed with 20 column volume of a binding buffer (1 M NaCl, 20 mM HEPES, pH 7.2) at a pressure of 1 ml/min. The reference extracellular vesicles separated in Example 1 were dissolved in an amount of 4 x 10¹⁰ vesicles in the same binding buffer.

Using the same binding buffer, the reference extracellular vesicle solution was loaded to the washed stationary phase having a hydrophobic functional group for 5 min, followed by washing out unbound residues with the same buffer for 5 min. Then, 20 column volumes with a concentration gradient of 1 M-0 M NaCl were applied before washing with 10 column volumes of 0 M NaCl buffer.

Eluates from the extracellular vesicle binding, washing, NaCl concentration gradient, and final washing procedures were fractioned by 1 ml. All of the eluate fractions were subjected to nanoparticle concentration analysis and sandwich ELISA based on anti-extracellular vesicle marker antibodies (anti-CD9 and anti-SW480EV antibody) to assay binding behaviors of the extracellular vesicles.

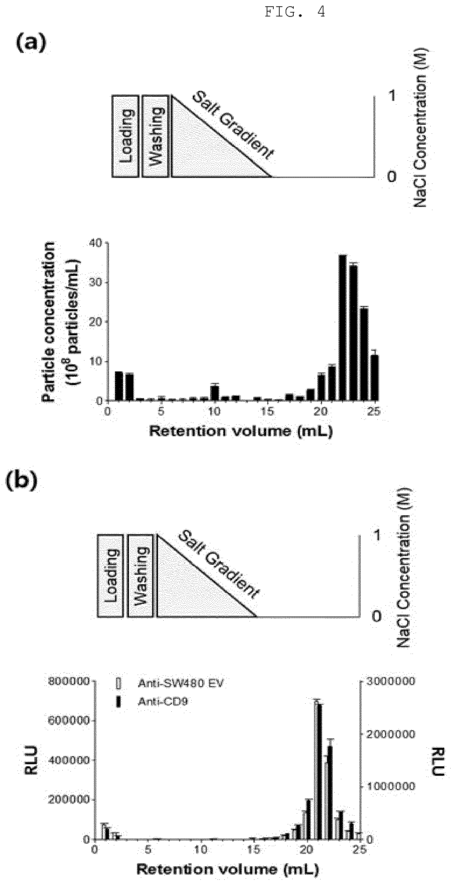

Results are depicted in FIG. 4. As shown in FIG. 4, most of the reference extracellular vesicles bound to the stationary phase having a hydrophobic function group and thus was not detected in the eluates under the environment of 1 M NaCl buffer. When the concentration of NaCl in the mobile phase reached 0 M, the elution of the extracellular vesicles was detected. Therefore, it is understood that the extracellular vesicles bind to the hydrophobic function group in a high-concentration NaCl environment whereas being separated from the hydrophobic function group in a low-concentration NaCl environment. In addition, the results obtained above indicate that a stationary phase having a hydrophobic functional group allows the effective elimination of impurities present in the purified reference extracellular vesicles, or inactivated extracellular vesicles or large residual impurity complexes formed during storage.

### EXAMPLE 3: Binding Behavior of Reference Extracellular Vesicle to Stationary Phase Having Hydrophobic Functional Group According to Salt Concentration

In order to examine the binding behavior of the reference extracellular vesicles to a stationary phase having a hydrophobic functional group according to NaCl concentrations, the following experiment was performed.

The purified reference extracellular vesicles were precipitated using polyethylene glycol. The precipitated reference extracellular vesicles were added to buffers with various concentrations of NaCl (0 M, 50 mM, 150 mM, 500 mM NaCl in 20 mM HEPES, pH7.2).

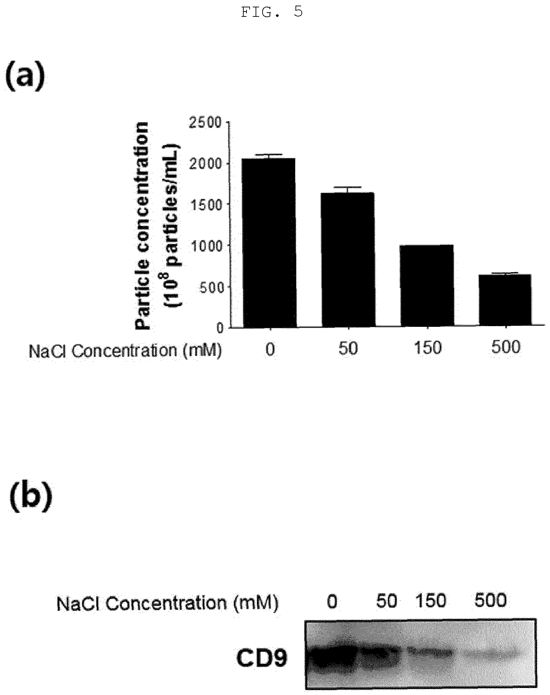

An empty column was filled with 0.4 ml of a stationary phase (Phenyl-Sepharose) having a hydrophobic functional group and then washed three times with 0.5 ml of water by centrifugation (700 xg, 1 min) to prepare hydrophobic interaction chromatography column. Four of such hydrophobic interaction chromatography columns were washed three times with 0.5 ml of respective buffers (0 M, 50 mM, 150 mM, 500 mM NaCl in 20 mM HEPES, pH7.2) for each time. To each of the prepared columns was loaded 0.2 ml of the reference extracellular vesicles, followed by reaction for 10 min. Centrifugation at 700 xg for 1 min discharged unbound vesicles. The solutions thus harvested were measured for nanoparticle concentrations to quantitate the reference extracellular vesicles therein, and Western blot analysis against CD9, which is a marker of extracellular vesicles, was performed. The results are given in FIG. 5.

As shown in both the nanoparticle analysis result (a) and the Western blots (b) of FIG. 5, the highest nanoparticle concentration and the most intensive CD9 signal were detected in the buffer with 0 M NaCl, indicating that the extracellular vesicles bind to the hydrophobic functional group in the condition of a high concentration of NaCl, but escape out of the column without being bound to the hydrophobic functional group in the condition of a low concentration of NaCl.

### EXAMPLE 4: Isolation of Blood-Derived Extracellular Vesicles Free of Lipoprotein by Hydrophobic Interaction Chromatography

Using the method of the present invention, extracellular vesicles were isolated from human plasma and a lipoprotein elimination effect was analyzed as follows.

An empty column was filled with 2 ml of a stationary phase (Phenyl-Sepharose) having a hydrophobic functional group and washed three times with 2 ml of water by centrifugation (700 xg, 1 min). Subsequently, 1 ml of EDTA-treated human plasma sample was loaded into the stationary phase-filled column which was then centrifuged again at 700 xg for 1 min to harvest the plasma sample that were not bound to the column. Thereafter, the harvested plasma sample was loaded again to the same column and centrifuged in the same condition. This procedure was performed three times in total.

The resulting plasma sample passing three times through the stationary phase-filled, hydrophobic interaction chromatography column was diluted eight times in a HEPES buffer (150 mM NaCl, HEPES, pH7.2) while a plasma sample that had not passed through the hydrophobic interaction chromatography column was used as a control. Using polyethylene glycol, extracellular vesicles in the samples were precipitated and centrifuged centrifugation (12,000 xg, 10 min). The precipitates of extracellular vesicles were collected and dissolved in HEPES buffer, followed by additional purification through S500 size exclusion chromatography using an HPLC system.

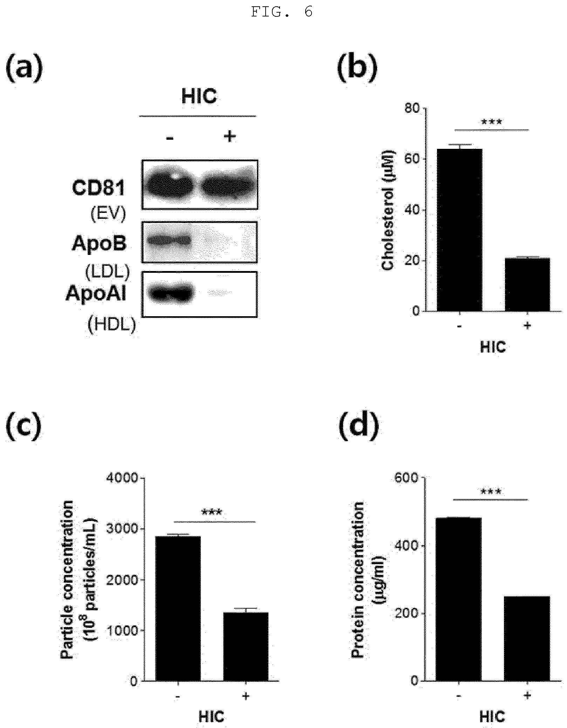

The blood-derived extracellular vesicles purified above were analyzed for CD81, which is a marker protein of extracellular vesicles, and ApoA1 and ApoB, which are proteins specific for high- and low-density lipoproteins, respectively, by Western blotting. The results are given in FIG. 6 (a). As is understood from the data, similar CD81 signals were detected in both the samples that passed and did not pass through the hydrophobic interaction chromatography column whereas no signals for ApoB and ApoA1, which are markers of lipoproteins, were detected from the sample that passed through the hydrophobic interaction chromatography column. Therefore, the results indicate that the use of a hydrophobic interaction chromatography column can selectively remove only lipoproteins while retaining the yield for the blood-derived extracellular vesicles.

In addition, the extracellular vesicle sample that passed through the hydrophobic interaction chromatography column was found to significantly decrease in cholesterol concentration as measured by an assay for cholesterol concentrations of the blood-derived extracellular vesicles purified above (FIG. 6(b)), as well as in G protein concentration as measured by a nanoparticle assay (FIG. 6(c) and a protein concentration assay (FIG. 6(d)), which accounts for the removal of lipoprotein nanoparticles.

Therefore, this Example demonstrates that the use of a hydrophobic interaction chromatography column can effectively prevent the contamination of blood-derived extracellular vesicles with lipoproteins, which are difficult to remove with conventional methods, while retaining the yield for the extracellular vesicles.

## Claims

1. A method for isolating extracellular vesicles, the method comprising the steps of:
(a) immobilizing a hydrophobic functional group onto a stationary phase;
(b) loading a sample containing extracellular vesicles to the stationary phase to bind the extracellular vesicles to the hydrophobic functional group;
(c) washing out remaining sample residues not bound to the hydrophobic functional group on the stationary phase; and
(d) eluting extracellular vesicles bound to the hydrophobic functional group from the stationary phase.

2. The method of claim 1, further comprising a step of washing the stationary phase having the hydrophobic functional group thereon with a solution containing salting-out ions, prior to step (b).

3. The method of claim 2, wherein the salting-out ions have a concentration suitable for controlling the hydrophobicity of the stationary phase to enable the extracellular vesicles to bind to the stationary phase.

4. The method of claim 3, wherein the concentration of the salting-out ions ranges from 0 M to 10 M.

5. The method of claim 1, further comprising a step of adding a solution containing salting-out ions to the sample to change the kind or concentration of salting-out ions contained in the sample, prior to step (b) of loading a sample containing extracellular vesicles to the stationary phase.

6. The method of claim 1, further comprising a step of controlling the kind or concentration of the salting-out ions to remove materials weaker in intensity of hydrophobic interaction than the extracellular vesicles, prior to step (d) of eluting the extracellular vesicles.

7. The method of claim 6, wherein the concentration of the salting-out ions for removing materials weaker in intensity of hydrophobic interaction than the extracellular vesicles is controlled to be within a range of 0 M to 10 M.

8. The method of claim 1, wherein the salting-out ions are at least one selected from the group consisting of sulfate, phosphate, hydroxide, fluoride, formate, acetate, chloride, bromide, nitrate, iodide, thiocyanate, citrate, and tartrate.

9. The method of claim 1, wherein the stationary phase is at least one selected from the group consisting of agarose beads, sepharose beads, sephadex beads, silica beads, magnetic beads, gold nanoparticles, silver nanoparticles, iron oxide nanoparticles, a nylon membrane, nitrocellulose membrane, a PVDF membrane, paper, plastic, glass, and a metal sensor chip.

10. The method of claim 1, wherein the hydrophobic functional group is at least one selected from the group consisting of butyl, cyanobutyl, octyl, phenyl, and diphenyl.

11. The method of claim 1, wherein the sample is at least one selected from the group consisting of a mammalian cell culture, a bacterial cell culture, a yeast culture, a tissue extract, a cancer tissue, serum, plasma, saliva, tear, nasal mucus, sweat, urine, feces, cerebrospinal fluid, ascite, amniotic fluid, bronchoalveolar lavage fluid, pleural effusion, semen, milk, dust, fresh water, seawater, soil, and fermented foods.

12. The method of claim 1, further comprising a step of pre-treating the sample containing the extracellular vesicles, prior to step (b) of loading the sample to the stationary phase.

13. The method of claim 12, wherein the step of pre-treating is conducted by a technique selected from centrifugation, ultracentrifugation, filtration, ultrafiltration, dialysis, desalting column chromatography, sonication, density gradient ultracentrifugation, size exclusion chromatography, ion exchange chromatography, affinity chromatography, precipitation, and an aqueous two-phase system.

14. The method of claim 1, further comprising a step of post-treating the extracellular vesicles in the eluate from step (d).

15. The method of claim 14, wherein the step of post-treating is conducted by a technique selected from centrifugation, ultracentrifugation, filtration, ultrafiltration, dialysis, desalting column chromatography, sonication, density gradient ultracentrifugation, size exclusion chromatography, ion exchange chromatography, affinity chromatography, precipitation, and an aqueous two-phase system.

16. Extracellular vesicles isolated by the method of any one of claims 1 to 15.

17. A method for isolating extracellular vesicles, the method comprising the steps of:
(a) immobilizing a hydrophobic functional group onto a stationary phase;
(b) loading a sample containing extracellular vesicles to the stationary phase; and
(c) collecting a sample fraction containing extracellular vesicles not bound to the hydrophobic functional group from the stationary phase.

18. The method of claim 17, further comprising a step of washing the stationary phase having hydrophobic functional group thereon with a solution containing salting-out ions, prior to step (b).

19. The method of claim 18, wherein the concentration of the salting-out ions ranges from 0 M to 10 M.

20. The method of claim 17, further comprising a step of adding a solution either containing or not containing salting-out ions to the sample to change kinds or concentrations of salting-out ions contained in the sample, prior to step (b) of loading a sample containing extracellular vesicles to the stationary phase.

21. The method of claim 20, wherein the concentration of the salting-out ions ranges from 0 M to 10 M.

22. The method of claim 17, wherein the salting-out ions are at least one selected from the group consisting of sulfate, phosphate, hydroxide, fluoride, formate, acetate, chloride, bromide, nitrate, iodide, thiocyanate, citrate, and tartrate.

23. The method of claim 17, wherein the stationary phase is at least one selected from the group consisting of agarose beads, sepharose beads, sephadex beads, silica beads, magnetic beads, gold nanoparticles, silver nanoparticles, iron oxide nanoparticles, a nylon membrane, nitrocellulose membrane, a PVDF membrane, paper, plastic, glass, and a metal sensor chip.

24. The method of claim 17, wherein the hydrophobic functional group is at least one selected from the group consisting of butyl, cyanobutyl, octyl, phenyl, and diphenyl.

25. The method of claim 17, wherein the sample is at least one selected from the group consisting of a mammalian cell culture, a bacterial cell culture, a yeast culture, a tissue extract, a cancer tissue, serum, plasma, saliva, tear, nasal mucus, sweat, urine, feces, cerebrospinal fluid, ascite, amniotic fluid, bronchoalveolar lavage fluid, pleural effusion, semen, milk, dust, fresh water, seawater, soil, and fermented foods.

26. The method of claim 17, further comprising a step of pre-treating the sample containing the extracellular vesicles, prior to step (b) of loading the sample to the stationary phase.

27. The method of claim 17, wherein the pre-treatment step is conducted by a technique selected from centrifugation, ultracentrifugation, filtration, ultrafiltration, dialysis, desalting column chromatography, sonication, density gradient ultracentrifugation, size exclusion chromatography, ion exchange chromatography, affinity chromatography, precipitation, and an aqueous two-phase system.

28. The method of claim 17, further comprising a step of post-treating the extracellular vesicles in the sample fraction collected from step (c).

29. The method of claim 17, wherein the post-treatment step is conducted by a technique selected from centrifugation, ultracentrifugation, filtration, ultrafiltration, dialysis, desalting column chromatography, sonication, density gradient ultracentrifugation, size exclusion chromatography, ion exchange chromatography, affinity chromatography, precipitation, and an aqueous two-phase system.

30. Extracellular vesicles isolated by the method of any one of claims 17 to 29.

31. A method for removing a material greater in intensity of hydrophobic interaction than extracellular vesicles from a sample containing the extracellular vehicles, the method comprising the steps of:
(a) immobilizing a hydrophobic functional group onto a stationary phase;
(b) loading a sample containing extracellular vesicles to the stationary phase having the hydrophobic functional group immobilized thereon; and
(c) collecting a sample fraction containing extracellular vesicles not bound to the hydrophobic functional group from the stationary phase.

32. The method of claim 31, further comprising a step of washing the stationary phase having the hydrophobic functional group immobilized thereon with a solution containing salting-out ions, prior to step (b).

33. The method of claim 32, wherein the concentration of the salting-out ions ranges from 0 M to 10 M.

34. The method of claim 30, further comprising a step of adding a solution containing salting-out ions to the sample to change the kind or concentrations of salting-out ions contained in the sample, prior to step (b) of loading a sample containing extracellular vesicles to the stationary phase.

35. The method of claim 34, wherein the concentration of the salting-out ions ranges from 0 M to 10 M.

36. The method of claim 31, wherein the material greater in intensity of hydrophobic interaction than extracellular vesicles is a lipoprotein.
